# EUROPEAN PATENT SPECIFICATION

(11) **EP 3 307 294 B1**
(45) Date of publication and mention of the grant of the patent: **18.12.2019**
(21) Application number: 16806775.9
(22) Date of filing: 03.06.2016
(51) Int. Cl.: A61K 36/8968, A61K 36/428, A61K 36/482, A61K 36/64, A61P 25/24, A61K 36/79, A61K 36/804, A61K 36/8945, A61K 36/899, A61K 36/9068, A61K 36/28, A61K 36/344, A61K 36/54, A61K 36/57, A61K 36/704, A61K 36/71, A61K 36/725, A61K 36/744

(54) **PHARMACEUTICAL COMPOSITION FOR TREATMENT OF DEPRESSION AND PREPARATION METHOD THEREOF**
PHARMAZEUTISCHE ZUSAMMENSETZUNG ZUR BEHANDLUNG VON DEPRESSION UND HERSTELLUNGSVERFAHREN DAFÜR
COMPOSITION PHARMACEUTIQUE POUR LE TRAITEMENT DE LA DÉPRESSION, ET PROCÉDÉ DE PRÉPARATION ASSOCIÉ

(30) Priority: 10.06.2015 AU 2015203074
(43) Date of publication of application: 18.04.2018
(73) Proprietor: Thisherb Health Pty Ltd., Southbank, Victoria 3006 (AU)
(72) Inventor: YU, Luping, Southbank Victoria 3006 (AU); WANG, Wencheng, Southbank Victoria 3006 (AU)
(74) Representative: Pohlman, Sandra M.
(86) International application number: PCT/CN2016/084639
(87) International publication number: WO 2016/197877

(56) References cited:
- CN-A- 1 682 989
- CN-A- 101 496 865
- CN-A- 105 664 107
- DATABASE TCM [Online] SIPO; 13 February 2008 (2008-02-13), Wang Bingqui: "A Chinese medicine composition for treating neurasthenia and its preparation method", XP002779947, Database accession no. CN-200610104276-A & CN 101 121 016 A (XIAMEN GUILONG INVEST MAN CO L [CN]) 13 February 2008 (2008-02-13)
- DATABASE TCM [Online] SIPO; 5 December 2007 (2007-12-05), Ling Jing; Zhang Xingxing: "A Chinese medicine preparation for treating depression", XP002779948, Database accession no. CN-200610027112-A & CN 101 081 278 A (JING LIN [CN]) 5 December 2007 (2007-12-05)
- DATABASE TCM [Online] SIPO; 12 November 2014 (2014-11-12), Liu Shulan: "A kind of Chinese patent medicine for treating postpartum depression", XP002779949, Database accession no. CN-102014000631039 & CN 104 352 973 A (LIU SHULAN) 18 February 2015 (2015-02-18)
- DATABASE WPI Week 201319 Thomson Scientific, London, GB; AN 2013-C71763 XP002779950, & CN 102 771 613 A (LI X) 14 November 2012 (2012-11-14)
- SHAFEEN S ET AL: "Evaluation of antianxiety and antidepressant activity of Cassia occidentalis leaves", ASIAN JOURNAL OF PHARMACEUTICAL AND CLINICAL RESEARCH 2012 ASIAN JOURNAL OF PHARMACEUTICAL AND CLINICAL RESEARCH IND, vol. 5, no. SUPPL. 3, 2012, pages 47-50, XP9504729, ISSN: 0974-2441

## Description

### FIELD OF THE INVENTION

The present invention relates to a compound preparation of traditional Chinese medicine, in particular to a pharmaceutical composition for use in treatment of Depression and the preparation method thereof.

### BACKGROUND OF THE INVENTION

Depression is a common emotional disorder which is a kind of sustained mood depression with the symptoms including impassiveness, sluggish thinking accompanied by loss of appetite, sexual dysfunction, sleep disorders and other physical symptoms. Depression can be caused by a variety of reasons, and the main clinical features of which are a significant and persistent depressed mood that is not commensurate with their situations. In serious cases, these may manifest into suicidal thoughts and behaviors. Most cases have a tendency to recur repeatedly. In majority of cases, each episode may be resolved. However, in some cases, there may have residual symptoms or the symptoms become chronic. Chinese medicine believes that Depression belongs to the category of Melancholia. It is a class of diseases caused by emotional stress, qi stagnation and unresolved despondency. It manifests mainly as depression, mood restlessness, flank pain, irritability, teary, insomnia and other complex symptoms.

At present, seven classes of antidepressants are used for the treatment of depression: serotonin reuptake inhibitors, monoamine oxidase inhibitors, phenyl-piperazine derivatives, serotonin - norepinephrine reuptake inhibitors, amino ketones, tricyclics, and four azepine ring piperazine. However, these synthetic antidepressants have a narrow spectrum, wide toxic side effects, high drug prices, ease of relapse and other disadvantages.

The symptoms of depression are complex including liver qi stagnation, mood swing and changeable temperament, mostly as a result of emotional instability. Its pathogenesis is mainly liver qi stagnation, heart and spleen lacking vitalities. Therefore, the medication used is directed to adjusting qi, expectorant, sedation, resuscitation, and heart tonification. Chinese herbal compounds have several features of multiple components, multitudinous activities and multiple targets. The multi-component synergies make up the low levels of the active ingredients and reduce the side effects thereof. Therefore, the unique characteristics of traditional Chinese herbal compounds render them as research hotspots for the development of a good and efficacious antidepressant with few side effects, safe and reliable for long-term use.

### SUMMARY OF THE INVENTION

The present invention discloses a pharmaceutical composition for the use in treatment of depression and its preparation method. The method is believed to be simple, safe and reliable, and suitable for industrial production.

In one aspect of the invention, the pharmaceutical composition for the use in treatment of Depression, consists of the following herbs in weight parts: 2-10 parts Ramulus Cinnamomi, 2-10 parts Rhizoma Zingiberis, 2-10 parts Fructus Schisandrae Chinensis, 2-8 parts Semen Ziziphi Spinosae, 2-8 parts Radix Codonopsis, 1-3 parts Rhizoma Dioscoreae, 0.5-2.5 parts Flos Inulae, 1-3 parts Caulis Bambusae in Taenia, 1-3 parts Fructus Trichosanthis, 1.5-4 parts Caulis Polygoni Multiflori, 1.5-4 parts Radix Ophiopogonis, 2-8 parts Radix Rehmanniae, 1.5-4 parts Cortex Moutan Radicis, 0.5-2.5 parts Fructus Gardeniae, 0.5-2.5 parts Herba Lophatheri, and 0.2-1.5 parts Folium Sennae.

Preferably, the Semen Ziziphi Spinosae is stir-fried by stir-frying the clean Semen Ziziphi Spinosae in a pot on low fire until the skin blisters and turns slightly yellow, removing and cooling. Semen Ziziphi Spinosae has properties of nourishing the liver, steadying the heart and nerves, and arresting sweating.

In one embodiment, the pharmaceutical composition is consisted of the following herbs in weight parts: 2-8 parts Ramulus Cinnamomi, 2-8 parts Rhizoma Zingiberis, 2-8 parts Fructus Schisandrae Chinensis, 2-6 parts Semen Ziziphi Spinosae, 2-6 parts Radix Codonopsis, 1-2 parts Rhizoma Dioscoreae, 0.5-1.5 parts Flos Inulae, 1-2 parts Caulis Bambusae in Taenia, 1-2 parts Fructus Trichosanthis, 1.5-3 parts Caulis Polygoni Multiflori, 1.5-3 parts Radix Ophiopogonis, 2-6 parts Radix Rehmanniae, 1.5-3 parts Cortex Moutan Radicis, 0.5-2 parts Fructus Gardeniae, 0.5-2 parts Herba Lophatheri, and 0.2-1 parts Folium Sennae. It is believed that the pharmaceutical composition having the raw materials in such ratios offers a high degree of safety and is effective for the use in treatment of depression.

In a preferred embodiment, the pharmaceutical composition is consisted of the following herbs in weight parts: 4 parts Ramulus Cinnamomi, 4 parts Rhizoma Zingiberis, 4 parts Fructus Schisandrae Chinensis, 3 parts Semen Ziziphi Spinosae, 3 parts Radix Codonopsis, 1.5 parts Rhizoma Dioscoreae, 1 part Flos Inulae, 1.5 parts Caulis Bambusae in Taenia, 1.5 parts Fructus Trichosanthis, 2 parts Caulis Polygoni Multiflori, 2 parts Radix Ophiopogonis, 3 parts Radix Rehmanniae, 2 parts Cortex Moutan Radicis, 1 part Fructus Gardeniae, 1 part Herba Lophatheri, and 0.5 parts Folium Sennae.

In a more preferred embodiment, the pharmaceutical composition per 35g is consisted of the following herbs in weight: 4g Ramulus Cinnamomi, 4g Rhizoma Zingiberis, 4g Fructus Schisandrae Chinensis, 3g Semen Ziziphi Spinosae, 3g Radix Codonopsis, 1.5g Rhizoma Dioscoreae, 1g Flos Inulae, 1.5g Caulis Bambusae in Taenia, 1.5g Fructus Trichosanthis, 2g Caulis Polygoni Multiflori, 2g Radix Ophiopogonis, 3g Radix Rehmanniae, 2g Cortex Moutan Radicis, 1g Fructus Gardeniae, 1g Herba Lophatheri, and 0.5g Folium Sennae.

Preferably, the pharmaceutical composition for the use in treatment of Depression is manufactured in the form of oral liquid, tablets, capsules, pills, dripping pills or granules.

Another aspect is directed to a method for preparing the pharmaceutical composition for the use in treatment of Depression comprises the following steps that:
A. All the 16 herbs are blended and boiling decocted by three times and the amount of water added in the decoction process is 7-10 times the total weight of the herbs, wherein the first decoction for 1.5-3h, the second decoction for 1-2h and the third decoction for 0.5-1.5h. When the amount of water added is 7-10 times the total weight of the herbs, the dissolution effects of the herbs will be better during the decoctions, and when the herbs are boiling decocted by three times, the extraction effects of the herbs will be more ideal;
B. The filtrates from the three decoctions are combined and concentrated into an extracting solution with relative density of 1.20-1.25; and
C. The extracting solution is added with ethanol to a concentration of 50-80%, standing for 24h, and the supernatant is removed and concentrated into an alcohol extract with relative density of 1.12-1.20 which is then dried to obtain the pharmaceutical composition.

In one embodiment, the method further comprises step D that the pharmaceutical composition is added with starch and homogenized before filling into capsules.

In one embodiment, the step B further comprises that after the filtrate is combined, the combined filtrate is concentrated into an extract with relative density of 1.20-1.25 at 60 °C.

In one embodiment, the step C comprises that the supernatant is concentrated into an extract relative density of 1.12-1.20 at 60 °C and spray-dried to obtain the pharmaceutical composition for the treatment of menopausal syndrome.

The present invention is a pharmaceutical composition prepared from traditional medicinal herbs of Ramulus Cinnamomi, Rhizoma Zingiberis, Fructus Schisandrae Chinensis, Semen Ziziphi Spinosae, Radix Codonopsis, Rhizoma Dioscoreae, Flos Inulae, Caulis Bambusae in Taenia, Fructus Trichosanthis, Caulis Polygoni Multiflori, Radix Ophiopogonis, Radix Rehmanniae, Cortex Moutan Radicis, Fructus Gardeniae, Herba Lophatheri and Folium Sennae. After clinical trials, all the 16 herbs have a synergistic effect with the following results: the pharmaceutical composition can nourish the liver and heart, improve the qi in spleen, clear heat and expectorant, and relieve emotional stress. The pharmaceutical composition is applicable for liver and spleen deficiencies, depression caused by melancholy, prevalent mental depression, emotional discomfort, sluggish thinking, insomnia, chest tightness, irritability, bloating, belching, lassitude, sadness, lack of appetite, constipation, pink tongue, weak pulse.

It is believed that the preparation method of the present invention is simple, environmental, economic, efficient, non-toxic and has the prospect of broad applications.

### EXAMPLES

### Example 1

A pharmaceutical composition for the use in treatment of depression is manufactured in form of oral liquid, in which the pharmaceutical composition is consisted of the following herbs in weight parts: 2 parts Ramulus Cinnamomi, 2 parts Rhizoma Zingiberis, 2 parts Fructus Schisandrae Chinensis, 2 parts Semen Ziziphi Spinosae, 2 parts Radix Codonopsis, 1 part Rhizoma Dioscoreae, 0.5 parts Flos Inulae, 1 part Caulis Bambusae in Taenia, 1 part Fructus Trichosanthis, 1.5 parts Caulis Polygoni Multiflori, 1.5 parts Radix Ophiopogonis, 2 parts Radix Rehmanniae, 1.5 parts Cortex Moutan Radicis, 0.5 part Fructus Gardeniae, 0.5 part Herba Lophatheri, and 0.2 parts Folium Sennae.

The method for preparing the pharmaceutical composition for the use in treatment of depression comprises the following steps that:
A. All the 16 herbs are blended and boiling decocted by three times and the amount of water added in the decoction process is 7 times of the total weight of the herbs, wherein the first decoction for 1.5h, the second decoction for 1h and the third decoction for 0.5h;
B. The filtrates from the three decoctions are combined and concentrated into an extracting solution with relative density of 1.20; and
C. The extracting solution is added with ethanol to a concentration of 50%, standing for 24h, and the supernatant is removed and concentrated into an alcohol extract with relative density of 1.12, and then blended and pelletized to obtain the pharmaceutical composition for the treatment of depression.

### Example 2

A pharmaceutical composition for the use in treatment of depression is manufactured in form of capsules, in which the pharmaceutical composition is consisted of the following herbs in weight parts: 10 parts Ramulus Cinnamomi, 10 parts Rhizoma Zingiberis, 10 parts Fructus Schisandrae Chinensis, 8 parts Semen Ziziphi Spinosae, 8 parts Radix Codonopsis, 3 parts Rhizoma Dioscoreae, 2.5 parts Flos Inulae, 3 parts Caulis Bambusae in Taenia, 3 parts Fructus Trichosanthis, 4 parts Caulis Polygoni Multiflori, 4 parts Radix Ophiopogonis, 8 parts Radix Rehmanniae, 4 parts Cortex Moutan Radicis, 2.5 parts Fructus Gardeniae, 2.5 parts Herba Lophatheri, and 1.5 parts Folium Sennae.

The method for preparing the pharmaceutical composition for the use in treatment of depression comprises the following steps that:
A. All the 16 herbs are blended and boiling decocted by three times and the amount of water added in the decoction process is 10 times of the total weight of the herbs, wherein the first decoction for 3h, the second decoction for 2h and the third decoction for 1.5h;
B. The filtrates from the three decoctions are combined and concentrated into an extracting solution with relative density of 1.20-1.25 at 60 °C;
C. The extracting solution is added with ethanol to a concentration of 80%, standing for 24h, and the supernatant is removed and concentrated into an alcohol extract with relative density of 1.20 at 60 °C, and then spray dried; and
D. Adding starch, blending and encapsulating to obtain the pharmaceutical composition for the treatment of depression.

The resulting capsule is a hard capsule and each capsule has 0.5g in weight. The content of the capsule is a brown powder with fragrant, sweet, and bitter. Four capsules are taken once, three times per day.

### Example 3

A pharmaceutical composition for the use in treatment of depression is manufactured in form of tablets, in which the pharmaceutical composition is consisted of the following herbs in weight parts: 8 parts Ramulus Cinnamomi, 8 parts Rhizoma Zingiberis, 8 parts Fructus Schisandrae Chinensis, 6 parts Semen Ziziphi Spinosae, 6 parts Radix Codonopsis, 2 parts Rhizoma Dioscoreae, 1.5 parts Flos Inulae, 2 parts Caulis Bambusae in Taenia, 2 parts Fructus Trichosanthis, 3 parts Caulis Polygoni Multiflori, 3 parts Radix Ophiopogonis, 6 parts Radix Rehmanniae, 3 parts Cortex Moutan Radicis, 2 parts Fructus Gardeniae, 2 parts Herba Lophatheri, and 1 part Folium Sennae.

The method for preparing the pharmaceutical composition for the use in treatment of depression comprises the following steps that:
A. All the 16 herbs are blended and boiling decocted by three times and the amount of water added in the decoction process is 8 times of the total weight of the herbs, wherein the first decoction for 2h, the second decoction for 1.5h and the third decoction for 1h;
B. The filtrates from the three decoctions are combined and concentrated into an extracting solution with relative density of 1.23 at 60 °C; and
C. The extracting solution is added with ethanol to a concentration of 60%, standing for 24h, and the supernatant is removed and concentrated into an alcohol extract with relative density of 1.15 at 60 °C, and then spray dried, granulated and compressed to obtain the pharmaceutical composition for the treatment of depression.

### Example 4

A pharmaceutical composition for use in treatment of menopausal syndrome is manufactured in form of pills, in which the pharmaceutical composition is consisted of the following herbs in weight parts: 4 parts Ramulus Cinnamomi, 4 parts Rhizoma Zingiberis, 4 parts Fructus Schisandrae Chinensis, 3 parts Semen Ziziphi Spinosae, 3 parts Radix Codonopsis, 1.5 parts Rhizoma Dioscoreae, 1 part Flos Inulae, 1.5 parts Caulis Bambusae in Taenia, 1.5 parts Fructus Trichosanthis, 2 parts Caulis Polygoni Multiflori, 2 parts Radix Ophiopogonis, 3 parts Radix Rehmanniae, 2 parts Cortex Moutan Radicis, 1 part Fructus Gardeniae, 1 part Herba Lophatheri, and 0.5 parts Folium Sennae.

The method for preparing the pharmaceutical composition for the use in treatment of depression comprises the following steps that:
A. All the 16 herbs are blended and boiling decocted by three times and the amount of water added in the decoction process is 8 times of the total weight of the herbs, wherein the first decoction for 2h, the second decoction for 1.5h and the third decoction for 1h;
B. The filtrates from the three decoctions are combined and concentrated into an extracting solution with relative density of 1.23 at 60 °C;
C. The extracting solution is added with ethanol to a concentration of 60%, standing for 24h, and the supernatant is removed and concentrated into an alcohol extract with relative density of 1.15 at 60 °C, and then blended and granulated to obtain the pharmaceutical composition for the treatment of depression.

### Example 5

A pharmaceutical composition for the use in treatment of depression is manufactured in form of granules, in which the pharmaceutical composition per 35g is consisted of the following herbs in weights: 4g Ramulus Cinnamomi, 4g Rhizoma Zingiberis, 4g Fructus Schisandrae Chinensis, 3g Semen Ziziphi Spinosae, 3g Radix Codonopsis, 1.5g Rhizoma Dioscoreae, 1g Flos Inulae, 1.5g Caulis Bambusae in Taenia, 1.5g Fructus Trichosanthis, 2g Caulis Polygoni Multiflori, 2g Radix Ophiopogonis, 3g Radix Rehmanniae, 2g Cortex Moutan Radicis, 1g Fructus Gardeniae, 1g Herba Lophatheri, and 0.5g Folium Sennae.

The method for preparing the pharmaceutical composition for the use in treatment of depression comprises the following steps that:
A. All the 16 herbs are blended and boiling decocted by three times and the amount of water added in the decoction process is 8 times of the total weight of the herbs, wherein the first decoction for 2h, the second decoction for 1.5h and the third decoction for 1h;
B. The filtrates from the three decoctions are combined and concentrated into an extracting solution with relative density of 1.23 at 60 °C; and
C. The extracting solution is added with ethanol to a concentration of 60%, standing for 24h, and the supernatant is removed and concentrated into an alcohol extract with relative density of 1.15 at 60 °C, and then dried and pelletized to obtain the pharmaceutical composition for the treatment of depression.

### Example 6

A pharmaceutical composition for use in treatment of depression is manufactured in form of dripping pills, in which the pharmaceutical composition is consisted of the following herbs in weight parts: 4g Ramulus Cinnamomi, 4g Rhizoma Zingiberis, 4g Fructus Schisandrae Chinensis, 3g Semen Ziziphi Spinosae, 3g Radix Codonopsis, 1.5g Rhizoma Dioscoreae, 1g Flos Inulae, 1.5g Caulis Bambusae in Taenia, 1.5g Fructus Trichosanthis, 2g Caulis Polygoni Multiflori, 2g Radix Ophiopogonis, 3g Radix Rehmanniae, 2g Cortex Moutan Radicis, 1g Fructus Gardeniae, 1g Herba Lophatheri, and 0.5g Folium Sennae.

The method for preparing the pharmaceutical composition for the use in treatment of depression comprises the following steps that:
A. All the 16 herbs are blended and boiling decocted by three times and the amount of water added in the decoction process is 8 times of the total weight of the herbs, wherein the first decoction for 2h, the second decoction for 1.5h and the third decoction for 1h;
B. The filtrates from the three decoctions are combined and concentrated into an extracting solution with relative density of 1.23 at 60 °C; and
C. The extracting solution is added with ethanol to a concentration of 60%, standing for 24h, and the supernatant is removed and concentrated into an alcohol extract with relative density of 1.15 at 60 °C, and then batched and made into dripping pills to obtain the pharmaceutical composition for the treatment of menopausal syndrome.

### Example 7

The following conclusions are the results of an experimental study on anti-depression effect by using capsules manufactured from the above pharmaceutical compositions.

### 1 Drugs and Materials

1.1 Drug testing and reagents: The capsules pharmaceutical compositions for the treatment of depression in form of obtained in Example 2 compared with fluoxetine hydrochloride.
1.2 Animals: 20 male and 20 female mice provided by the Experimental Animal Center; average body mass of 18∼22g.
1.3 Instruments: Mouse-tail suspension device (a wooden board of about 1 meter in length, fixed onto a desktop with a horizontal distance of about 15cm), swimming model device (a glass tank with a height of 30cm, a diameter of 14cm, a water depth of 10cm, and temperature of 25 °C), and homemade open box.
1.4 Grouping: The mice were fed for one week, during which time the mice feed and drink freely. The mice were randomly divided into four groups of control group, depression capsule low-dose group (hereinafter referred to as the low-dose group), depression capsule high-dose group (hereinafter referred to as the high-dose group), the positive groups, 10 mice per group.
1.5 Drug administration: The control group was given saline daily at 0.2ml / 10g; the positive control group was given fluoxetine hydrochloride 2.4mg / kg; the low-dose group was given depression capsules 1.05g / kg; and the high-dose group was given depression capsules 4.20g / kg. The route of administration was directly into the stomach.

### 1.6 Methods

### 1.6.1 The mouse-tail suspension test: Refer to the method by Stern et al.

The mouse was suspended by its tail so that the tail was adhered (1cm from the tail end of the mouse) to the horizontal plank of wooden board by tape with its head about 5cm above the desktop. A cardboard was set on each side of the horizontal plank to block out the sight of the mouse. The mouse struggled to overcome the abnormal postural, but it ceased to move intermittently after a while, which shows a state of disappointment. The test was performed in 1 h after last drug administration and the time of the mouse being immobile within last 4 min for 6 min period was recorded.

### 1.6.2 Mouse forced swimming test: Refer to method by Prosolt et al.

A tank is provided with 30cm height, 14cm diameter, water depth 10cm, and water temperature 25 °C. The test was performed in 1 h after last drug administration. The mouse was placed in water for 6min and the accumulated time of the mouse being immobile within last 4 min. The immobility is defined as the mouse does not move or struggle in the water or be floating state on water while only small limb movements occur in order to keep the head surfaced.

### 1.6.3 Opening test: Refer to method by Elliott et al.

A wooden box was provided with 36cm × 36cm × 27cm, the bottom of which is divided into 25 squares. The test was performed in 1 h after last drug administration, and the number of the mouse crossing the grids and vertical climbing within last 3 min for 5min period was recorded.

### 2 Results

2.1 Comparing the effects of the depression capsule on the immobile time in mouse-tail suspension test with the control group, the low-dose group has a significant difference (P <0.05), the high-dose group has very significant difference (P <0.01), and the positive group has highly significant difference (P <0.01). The results are shown in Table 1.

**Table 2: Effects of the capsule on the mouse immobile time in the mouse-tail suspension test (x̅ ±s, n=10)**

| Group | Dose (g / kg) | Immobile time in the mouse-tail suspension test (s) |
|---|---|---|
| Control group | - | 115.5 ± 30.7 |
| Low-dose group | 1.05 | 88.6 ± 23.8 * |
| High dose group | 4.20 | 74.2 ± 27.6 ** |
| Positive control group | 0.0024 | 73 ± 29.4 ** |

| | | |
|---|---|---|
| Note: Compared with the control group, ** P <0.01, * P <0.05. | | |

2.2 Comparing the effects of the capsule on the immobile time in the mouse forced swimming test with the control group, the low-dose group has a significant difference (P <0.05), the high-dose group has very significant difference (P <0.01), and the positive group has highly significant difference (P <0.01). The results are shown in Table 2.

**Table 2. Effects of the capsules on the mouse immobile time in the mouse forced swimming test (x̅ ±s, n=10)**

| Group | Dose (g / kg) | Immobile time in mouse forced swimming test (s) |
|---|---|---|
| Control group | - | 130.8 ± 22.6 |
| Low-dose group | 1.05 | 99.5 ± 37.1 * |
| High dose group | 4.20 | 71.2 ± 26.5 ** |
| Positive control group | 0.0024 | 83.5 ± 41.6 ** |

| | | |
|---|---|---|
| Note: Compared with the control group, ** P <0.01, * P <0.05. | | |

2.3 Comparing the effects of the depression capsule on the independent activity in the opening test with the control group, the low-dose group, high-dose group and positive group have no significant difference (P> 0.05). The results are shown in Table 3.

**Table 3 Effects of the capsule on the mouse independent activity in the opening test (± s, n = 10)**

| Group | Dose (g / kg) | the number of squares crossed by mouse |
|---|---|---|
| Control group | - | 41.5 ± 19.4 |
| Low-dose group | 1.05 | 42.1 ± 15.7 |
| High dose group | 4.20 | 47.2 ± 15.3 |
| Positive control group | 0.0024 | 43.9 ± 13.3 |

| | | |
|---|---|---|
| Note: Compared with the control group, P> 0.05. | | |

In summary, the above descriptions are only examples of the preferred embodiments of the present invention.

The reference to any prior art in this specification is not, and should not be taken as, an acknowledgement of any form of suggestion that such prior art forms part of the common general knowledge.

It will be understood that the term "comprise" and any of its derivatives (eg comprises, comprising) as used in this specification is to be taken to be inclusive of features to which it refers, and is not meant to exclude the presence of any additional features unless otherwise stated or implied.

## Claims

1. A pharmaceutical composition for use in the treatment of Depression, wherein the pharmaceutical composition consists of the following herbs in weight parts:
| | |
|---|---|
| Ramulus Cinnamomi | 2-10 parts, |
| Rhizoma Zingiberis | 2-10 parts, |
| Fructus Schisandrae Chinensis | 2-10 parts, |
| Semen Ziziphi Spinosae | 2-8 parts, |
| Radix Codonopsis | 2-8 parts, |
| Rhizoma Dioscoreae | 1-3 parts, |
| Flos Inulae, | 0.5-2.5 parts, |
| Caulis Bambusae in Taenia | 1-3 parts, |
| Fructus Trichosanthis | 1-3 parts, |
| Caulis Polygoni Multiflori | 1.5-4 parts, |
| Radix Ophiopogonis | 1.5-4 parts, |
| Radix Rehmanniae | 2-8 parts, |
| Cortex Moutan Radicis | 1.5-4 parts, |
| Fructus Gardeniae | 0.5-2.5 parts, |
| Herba Lophatheri | 0.5-2.5 parts, and |
| Folium Sennae | 0.2-1.5 parts. |

2. The pharmaceutical composition for use according to claim 1, wherein the Semen Ziziphi Spinosae is stir-fried.

3. The pharmaceutical composition for use according to claim 1, wherein the pharmaceutical composition consists of the following herbs in weight parts:
| | |
|---|---|
| Ramulus Cinnamomi | 2-8 parts, |
| Rhizoma Zingiberis | 2-8 parts, |
| Fructus Schisandrae Chinensis | 2-8 parts, |
| Semen Ziziphi Spinosae | 2-6 parts, |
| Radix Codonopsis | 2-6 parts, |
| Rhizoma Dioscoreae | 1-2 parts, |
| Flos Inulae | 0.5-1.5 parts, |
| Caulis Bambusae in Taenia | 1-2 parts, |
| Fructus Trichosanthis | 1-2 parts, |
| Caulis Polygoni Multiflori | 1.5-3 parts, |
| Radix Ophiopogonis | 1.5-3 parts, |
| Radix Rehmanniae | 2-6 parts, |
| Cortex Moutan Radicis | 1.5-3 parts, |
| Fructus Gardeniae | 0.5-2 parts, |
| Herba Lophatheri | 0.5-2 parts, and |
| Folium Sennae | 0.2-1 parts. |

4. The pharmaceutical composition for use according to claim 1, wherein the pharmaceutical composition consists of the following herbs in weight parts:
| | |
|---|---|
| Ramulus Cinnamomi | 4 parts, |
| Rhizoma Zingiberis | 4 parts, |
| Fructus Schisandrae Chinensis | 4 parts, |
| Semen Ziziphi Spinosae | 3 parts, |
| Radix Codonopsis | 3 parts, |
| Rhizoma Dioscoreae | 1.5 parts, |
| Flos Inulae | 1 part, |
| Caulis Bambusae in Taenia | 1.5 parts, |
| Fructus Trichosanthis | 1.5 parts, |
| Caulis Polygoni Multiflori | 2 parts, |
| Radix Ophiopogonis | 2 parts, |
| Radix Rehmanniae | 3 parts, |
| Cortex Moutan Radicis | 2 parts, |
| Fructus Gardeniae | 1 part, |
| Herba Lophatheri | 1 part, and |
| Folium Sennae | 0.5 parts. |

5. The pharmaceutical composition for use according to claim 1, wherein the pharmaceutical composition per 35g consists of the following herbs in weights:
| | |
|---|---|
| Ramulus Cinnamomi | 4 g, |
| Rhizoma Zingiberis | 4 g, |
| Fructus Schisandrae Chinensis | 4 g, |
| Semen Ziziphi Spinosae | 3 g, |
| Radix Codonopsis | 3 g, |
| Rhizoma Dioscoreae | 1.5 g, |
| Flos Inulae | 1 g, |
| Caulis Bambusae in Taenia | 1.5 g, |
| Fructus Trichosanthis | 1.5 g, |
| Caulis Polygoni Multiflori | 2 g, |
| Radix Ophiopogonis | 2 g, |
| Radix Rehmanniae | 3 g, |
| Cortex Moutan Radicis | 2 g, |
| Fructus Gardeniae | 1 g, |
| Herba Lophatheri | 1 g, and |
| Folium Sennae | 0.5 g. |

6. The pharmaceutical composition for use according to any one of claims 1-5, wherein the pharmaceutical composition is in the form of an oral liquid, tablets, capsules, granules, pills, or dripping pills.

7. A method for preparing the pharmaceutical composition for use according to any one of claims 1-5, comprising the steps that:
A.Blending all the 16 herbs and boiling decocted by three times and the amount of water added in the decoction process is 7-10 times the total weight of the herbs, wherein the first decoction for 1.5-3h, the second decoction for 1-2h and the third decoction for 0.5-1.5h;
B.Combining the filtrates from the three decoctions and concentrating into an extracting solution with relative density of 1.20-1.25; and
C. Adding ethanol to the extracting solution to a concentration of 50-80%, standing for 24h, removing the supernatant and concentrating into an alcohol extract with relative density of 1.12-1.20, and then drying to obtain the pharmaceutical composition.

8. The method according to claim 7, further comprising step D of adding starch to the pharmaceutical composition, and homogenizing the mixture and filling into capsules.

9. The method according to claim 7, wherein in step B the combined filtrates are at 60 °C and are concentrated to an extract of relative density of 1.20-1.25.

10. The method according to claim 7, wherein the step C further comprises concentrating the supernatant at 60 °C to an extract of relative density of 1.12-1.20 and then spray drying to obtain the pharmaceutical composition.

11. The pharmaceutical composition for use according to any one of claims 1-6 for use in a method of treating Depression in a subject, wherein the method comprises administering the pharmaceutical composition to a subject.

## Patentansprüche

1. Pharmazeutische Zusammensetzung zur Verwendung bei der Behandlung von Depression, wobei die pharmazeutische Zusammensetzung aus den folgenden Kräutern in Gewichtsteilen besteht:
| | |
|---|---|
| Ramulus Cinnamomi | 2 bis 10 Teile, |
| Rhizoma Zingiberis | 2 bis 10 Teile, |
| Fructus Schisandrae Chinensis | 2 bis 10 Teile, |
| Semen Ziziphi Spinosae | 2 bis 8 Teile, |
| Radix Codonopsis | 2 bis 8 Teile, |
| Rhizoma Dioscoreae | 1 bis 3 Teile, |
| Flos Inulae | 0,5 bis 2,5 Teile, |
| Caulis Bambusae in Taenia | 1 bis 3 Teile, |
| Fructus Trichosanthis | 1 bis 3 Teile, |
| Caulis Polygoni Multiflori | 1,5 bis 4 Teile, |
| Radix Ophiopogonis | 1,5 bis 4 Teile, |
| Radix Rehmanniae | 2 bis 8 Teile, |
| Cortex Moutan Radicis | 1,5 bis 4 Teile, |
| Fructus Gardeniae | 0,5 bis 2,5 Teile, |
| Herba Lophatheri | 0,5 bis 2,5 Teile und |
| Folium Sennae | 0,2 bis 1,5 Teile. |

2. Pharmazeutische Zusammensetzung zur Verwendung nach Anspruch 1, wobei der Semen Ziziphi Spinosae rührgegart wird.

3. Pharmazeutische Zusammensetzung zur Verwendung nach Anspruch 1, wobei die pharmazeutische Zusammensetzung aus den folgenden Kräutern in Gewichtsteilen besteht:
| | |
|---|---|
| Ramulus Cinnamomi | 2 bis 8 Teile, |
| Rhizoma Zingiberis | 2 bis 8 Teile, |
| Fructus Schisandrae Chinensis | 2 bis 8 Teile, |
| Semen Ziziphi Spinosae | 2 bis 6 Teile, |
| Radix Codonopsis | 2 bis 6 Teile, |
| Rhizoma Dioscoreae | 1 bis 2 Teile, |
| Flos Inulae | 0,5 bis 1,5 Teile, |
| Caulis Bambusae in Taenia | 1 bis 2 Teile, |
| Fructus Trichosanthis | 1 bis 2 Teile, |
| Caulis Polygoni Multiflori | 1,5 bis 3 Teile, |
| Radix Ophiopogonis | 1,5 bis 3 Teile, |
| Radix Rehmanniae | 2 bis 6 Teile, |
| Cortex Moutan Radicis | 1,5 bis 3 Teile, |
| Fructus Gardeniae | 0,5 bis 2 Teile, |
| Herba Lophatheri | 0,5 bis 2 Teile und |
| Folium Sennae | 0,2 Teile bis 1 Teil. |

4. Pharmazeutische Zusammensetzung zur Verwendung nach Anspruch 1, wobei die pharmazeutische Zusammensetzung aus den folgenden Kräutern in Gewichtsteilen besteht:
| | |
|---|---|
| Ramulus Cinnamomi | 4 Teile, |
| Rhizoma Zingiberis | 4 Teile, |
| Fructus Schisandrae Chinensis | 4 Teile, |
| Semen Ziziphi Spinosae | 3 Teile, |
| Radix Codonopsis | 3 Teile, |
| Rhizoma Dioscoreae | 1,5 Teile, |
| Flos Inulae | 1 Teil, |
| Caulis Bambusae in Taenia | 1,5 Teile, |
| Fructus Trichosanthis | 1,5 Teile, |
| Caulis Polygoni Multiflori | 2 Teile, |
| Radix Ophiopogonis | 2 Teile, |
| Radix Rehmanniae | 3 Teile, |
| Cortex Moutan Radicis | 2 Teile, |
| Fructus Gardeniae | 1 Teil, |
| Herba Lophatheri | 1 Teil und |
| Folium Sennae | 0,5 Teile. |

5. Pharmazeutische Zusammensetzung zur Verwendung nach Anspruch 1, wobei die pharmazeutische Zusammensetzung pro 35 g aus den folgenden Kräutern in Gewichten besteht:
| | |
|---|---|
| Ramulus Cinnamomi | 4 g, |
| Rhizoma Zingiberis | 4 g, |
| Fructus Schisandrae Chinensis | 4 g, |
| Semen Ziziphi Spinosae | 3 g, |
| Radix Codonopsis | 3 g, |
| Rhizoma Dioscoreae | 1,5 g, |
| Flos Inulae | 1 g, |
| Caulis Bambusae in Taenia | 1,5 g, |
| Fructus Trichosanthis | 1,5 g, |
| Caulis Polygoni Multiflori | 2 g, |
| Radix Ophiopogonis | 2 g, |
| Radix Rehmanniae | 3 g, |
| Cortex Moutan Radicis | 2 g, |
| Fructus Gardeniae | 1 g, |
| Herba Lophatheri | 1 g und |
| Folium Sennae | 0,5 g. |

6. Pharmazeutische Zusammensetzung zur Verwendung nach einem der Ansprüche 1 bis 5, wobei die pharmazeutische Zusammensetzung in der Form einer oralen Flüssigkeit, von Tabletten, Kapseln, Granulaten, Pillen oder Tropfpillen vorliegt.

7. Verfahren zum Herstellen der pharmazeutischen Zusammensetzung zur Verwendung nach einem der Ansprüche 1 bis 5, umfassend die folgenden Schritte:
A. Mischen aller 16 Kräuter und dreimaliges Abkochen, wobei die Menge des Wassers, das bei dem Abkochprozess zugegeben wird, das 7- bis 10-fache des Gesamtgewichts der Kräuter beträgt, wobei das erste Abkochen 1,5 bis 3 h, das zweite Abkochen 1 bis 2 h und das dritte Abkochen 0,5 bis 1,5 h andauert;
B. Kombinieren der Filtrate der drei Abkochungen und Konzentrieren zu einer Extraktionslösung mit einer relativen Dichte von 1,20 bis 1,25; und
C. Zugeben von Ethanol zu der Extraktionslösung mit einer Konzentration von 50 bis 80 %, Stehenlassen für 24 h, Entfernen des Überstands und Konzentrieren zu einem Alkoholextrakt mit einer relativen Dichte von 1,12 bis 1,20 und dann Trocknen, um die pharmazeutische Zusammensetzung zu erhalten.

8. Verfahren nach Anspruch 7, ferner umfassend Schritt D des Zugebens von Stärke zu der pharmazeutischen Zusammensetzung und Homogenisieren der Mischung und Abfüllen in Kapseln.

9. Verfahren nach Anspruch 7, wobei in Schritt B die Filtrate bei 60 °C kombiniert werden und zu einem Extrakt einer relativen Dichte von 1,20 bis 1,25 konzentriert werden.

10. Verfahren nach Anspruch 7, wobei der Schritt C ferner ein Konzentrieren des Überstands bei 60 °C zu einem Extrakt einer relativen Dichte von 1,12 bis 1,20 und dann ein Sprühtrocknen umfasst, um die pharmazeutische Zusammensetzung zu erhalten.

11. Pharmazeutische Zusammensetzung zur Verwendung nach einem der Ansprüche 1 bis 6 zur Verwendung bei einem Verfahren zum Behandeln von Depression bei einem Patienten, wobei das Verfahren ein Verabreichen der pharmazeutischen Zusammensetzung an einen Patienten umfasst.

## Revendications

1. Composition pharmaceutique pour utilisation dans le traitement de la dépression, dans laquelle la composition pharmaceutique est constituée des herbes suivantes en parties en poids :
| | |
|---|---|
| Ramulus Cinnamomi | 2 à 10 parties, |
| Rhizoma Zingiberis | 2 à 10 parties, |
| Fructus Schisandrae Chinensis | 2 à 10 parties, |
| Semen Ziziphi Spinosae | 2 à 8 parties, |
| Radix Codonopsis | 2 à 8 parties, |
| Rhizoma Dioscoreae | 1 à 3 parties, |
| Flos Inulae, | 0,5 à 2,5 parties, |
| Caulis Bambusae in Taenia | 1 à 3 parties, |
| Fructus Trichosanthis | 1 à 3 parties, |
| Caulis Polygoni Multiflori | 1,5 à 4 parties, |
| Radix Ophiopogonis | 1,5 à 4 parties, |
| Radix Rehmanniae | 2 à 8 parties, |
| Cortex Moutan Radicis | 1,5 à 4 parties, |
| Fructus Gardeniae | 0,5 à 2,5 parties, |
| Herba Lophatheri | 0,5 à 2,5 parties, et |
| Folium Sennae | 0,2 à 1,5 parties. |

2. Composition pharmaceutique pour utilisation selon la revendication 1, dans laquelle le Semen Ziziphi Spinosae est sauté.

3. Composition pharmaceutique pour utilisation selon la revendication 1, dans laquelle la composition pharmaceutique est constituée des herbes suivantes en parties en poids :
| | |
|---|---|
| Ramulus Cinnamomi | 2 à 8 parties, |
| Rhizoma Zingiberis | 2 à 8 parties, |
| Fructus Schisandrae Chinensis | 2 à 8 parties, |
| Semen Ziziphi Spinosae | 2 à 6 parties, |
| Radix Codonopsis | 2 à 6 parties, |
| Rhizoma Dioscoreae | 1 à 2 parties, |
| Flos Inulae | 0,5 à 1,5 parties, |
| Caulis Bambusae in Taenia | 1 à 2 parties, |
| Fructus Trichosanthis | 1 à 2 parties, |
| Caulis Polygoni Multiflori | 1,5 à 3 parties, |
| Radix Ophiopogonis | 1,5 à 3 parties, |
| Radix Rehmanniae | 2 à 6 parties, |
| Cortex Moutan Radicis | 1,5 à 3 parties, |
| Fructus Gardeniae | 0,5 à 2 parties, |
| Herba Lophatheri | 0,5 à 2 parties, et |
| Folium Sennae | 0,2 à 1 partie. |

4. Composition pharmaceutique pour utilisation selon la revendication 1, dans laquelle la composition pharmaceutique est constituée des herbes suivantes en parties en poids :
| | |
|---|---|
| Ramulus Cinnamomi | 4 parties, |
| Rhizoma Zingiberis | 4 parties, |
| Fructus Schisandrae Chinensis | 4 parties, |
| Semen Ziziphi Spinosae | 3 parties, |
| Radix Codonopsis | 3 parties, |
| Rhizoma Dioscoreae | 1,5 parties, |
| Flos Inulae | 1 partie, |
| Caulis Bambusae in Taenia | 1,5 parties, |
| Fructus Trichosanthis | 1,5 parties, |
| Caulis Polygoni Multiflori | 2 parties, |
| Radix Ophiopogonis | 2 parties, |
| Radix Rehmanniae | 3 parties, |
| Cortex Moutan Radicis | 2 parties, |
| Fructus Gardeniae | 1 partie, |
| Herba Lophatheri | 1 partie, et |
| Folium Sennae | 0,5 parties. |

5. Composition pharmaceutique pour utilisation selon la revendication 1, dans laquelle la composition pharmaceutique par 35 g est constituée des herbes suivantes en poids :
| | |
|---|---|
| Ramulus Cinnamomi | 4 g, |
| Rhizoma Zingiberis | 4 g, |
| Fructus Schisandrae Chinensis | 4 g, |
| Semen Ziziphi Spinosae | 3 g, |
| Radix Codonopsis | 3 g, |
| Rhizoma Dioscoreae | 1,5 g, |
| Flos Inulae | 1 g, |
| Caulis Bambusae in Taenia | 1,5 g, |
| Fructus Trichosanthis | 1,5 g, |
| Caulis Polygoni Multiflori | 2 g, |
| Radix Ophiopogonis | 2 g, |
| Radix Rehmanniae | 3 g, |
| Cortex Moutan Radicis | 2 g, |
| Fructus Gardeniae | 1 g, |
| Herba Lophatheri | 1 g, et |
| Folium Sennae | 0,5 g. |

6. Composition pharmaceutique pour utilisation selon l'une quelconque des revendications 1 à 5, dans laquelle la composition pharmaceutique est sous la forme d'un liquide oral, de comprimés, de gélules, de granules, de pilules, ou de pilules formées par egouttage.

7. Procédé de préparation de la composition pharmaceutique pour utilisation selon l'une quelconque des revendications 1 à 5, comprenant les étapes consistant à :
A. mélanger toutes les 16 herbes et faire bouillir en décoction par trois fois et la quantité d'eau ajoutée dans le processus de décoction vaut 7 à 10 fois le poids total des herbes, dans lequel la première décoction dure 1,5 à 3 h, la deuxième décoction dure 1 à 2 h et la troisième décoction dure 0,5 à 1,5 h ;
B. combiner les filtrats provenant des trois décoctions et concentrer dans une solution d'extraction avec une densité relative de 1,20 à 1,25 ; et
C. ajouter de l'éthanol à la solution d'extraction jusqu'à une concentration de 50 à 80 %, laisser reposer pendant 24 h, éliminer le surnageant et concentrer dans un extrait d'alcool avec une densité relative de 1,12 à 1,20, puis sécher pour obtenir la composition pharmaceutique.

8. Procédé selon la revendication 7, comprenant en outre une étape D d'ajout d'amidon à la composition pharmaceutique, et d'homogénéisation du mélange et de remplissage dans des gélules.

9. Procédé selon la revendication 7, dans lequel à l'étape B les filtrats combinés sont à 60 °C et sont concentrés jusqu'à un extrait d'une densité relative de 1,20 à 1,25.

10. Procédé selon la revendication 7, dans lequel l'étape C comprend en outre la concentration du surnageant à 60 °C jusqu'à un extrait d'une densité relative de 1,12 à 1,20 puis le séchage par atomisation pour obtenir la composition pharmaceutique.

11. Composition pharmaceutique pour utilisation selon l'une quelconque des revendications 1 à 6 pour utilisation dans un procédé de traitement de la dépression chez un sujet, dans lequel le procédé comprend l'administration de la composition pharmaceutique à un sujet.
